# EUROPEAN PATENT APPLICATION

(11) **EP 1 403 252 A1**
(43) Date of publication of application: **31.03.2004**
(21) Application number: 02736149.2
(22) Date of filing: 18.06.2002
(51) Int. Cl.: C07D 213/75, A61K 31/4409, A61P 7/02, A61P 9/10, A61P 29/00, A61P 31/04, A61P 35/04, A61P 37/06, A61P 43/00, C07C 69/76, C07C 69/734

(54) **NOVEL BUTADIENE DERIVATIVES, PROCESS FOR PREPARATION OF THE SAME AND INTERMEDIATES FOR THE SYNTHESIS THEREOF**

(30) Priority: 03.07.2001 JP 2001201686
(71) Applicant: Tanabe Seiyaku Co., Ltd., Chuo-ku Osaka 541-8505 (JP)
(72) Inventor: OHTANI, Akio, Kawaguchi-shi, Saitama 332-0034 (JP); OGIKU, Tsuyoshi, Kawaguchi-shi, Saitama 332-0017 (JP); YAMADA, Yasuhiro, Nakano-ku, Tokyo 164-0002 (JP)
(74) Representative: Hayes, Adrian Chetwynd
(86) International application number: PCT/JP2002/006047
(87) International publication number: WO 2003/004473

(57) **Abstract**

A butadiene derivative having an excellent inhibitory activity on the PAI-1, which is represented by the general formula [I]: 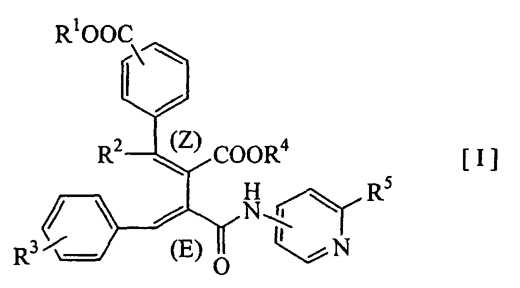 wherein R¹ is a hydrogen atom or a lower alkyl group, R² is a lower alkyl group, R³ is a lower alkoxy group, R⁴ is a hydrogen atom or a lower alkyl group, R⁵ is a lower alkyl group, or a pharmaceutically acceptable salt thereof, a process a process for preparing the same and an intermediate thereof.

## Description

### TECHNICAL FIELD

The present invention relates to a novel butadiene derivative, a process for preparing the same and intermediates for the synthesis thereof.

### BACKGROUND ART

The fibrinolysis (fibrinolytic system) plays an important role in the living body in the lysis of thrombus, the destruction and repair of tissue, the migration of cells, and the like. The fibrinolysis is activated when plasminogen is converted into plasmin by a plasminogen activator (PA). Type I plasminogen activator inhibitor (PAI-1) controls the fibrinolysis at the stage of start, and is construed as participating into the formation and development of various pathologies such as thrombosis, diabetes, arteriosclerosis, and the like. Accordingly, a compound capable of inhibiting the production of PAI-1 can be useful in the treatment or prophylaxis of thrombosis, diabetic complications, arteriosclerosis, and the like.

On the other hand, EP-A-563798 discloses as an antithrombotic agent (1E, 3E)-1-(3,4,5-trimethoxyphenyl)-2-methoxycarbonyl-3-[N-(4-pyridylmethyl)carbamoyl]-4-phenylbutadine and the like; however, it does not specifically disclose compounds having a 2-lower alkyl-pyridyl group as a substituent on the carbamoyl group attached to the 3-position of butadiene and, furthermore, is silent about compounds having a carboxyl group on the benzene ring attached to the 1-position of butadiene. In addition, the compounds disclosed in EP-A-563798 can stand further improvement in terms of bioavailability and stability.

WO97/36864 discloses (1Z,3E)-1-methyl-1-(3,5-dimethoxyphenyl)-2-methoxycarbonyl-3-[N-(4-pyridyl)aminocarbonyl]-4-(3,4-methylenedioxyphenyl)-butadiene, (1Z,3E)-1-methyl-1-(3,4,5-trimethoxyphenyl)-2-methoxycarbonyl-3-[N-(4-pyridyl)aminocarbonyl]-4-(3,4-methylenedioxyphenyl)butadiene, and the like; however, it does not disclose specifically compounds having a carboxyl group on the benzene ring attached to the 1-position of butadiene or those having a 2-lower alkyl-pyridyl group as a substituent on the carbamoyl group attached to the 3-position of butadiene, which are herein disclosed.

### DISCLOSURE OF THE INVENTION

The present invention provides a novel butadiene derivative, a process for preparing the same and an intermediate thereof, which butadiene derivative is highly safe as a medicament.

The present inventors have intensively studied and have found that a novel butadiene derivative wherein 1) the butadiene skeleton has a steric structure of (1Z, 3E); 2) the benzene ring binding at the 1-position of butadiene skeleton comprises a carboxyl group; and 3) the carbamoyl group binding at the 3-position of butadiene skeleton is substituted by a 2-lower alkyl-pyridyl group, inhibits the production of PAI-1, and has beneficial activities such as antithrombotic activity and is excellent in bioavailability, stability and less toxicity, and the like, and have finally accomplished the present invention.

Thus, the present invention provides a novel butadiene derivative of the general formula [I]: 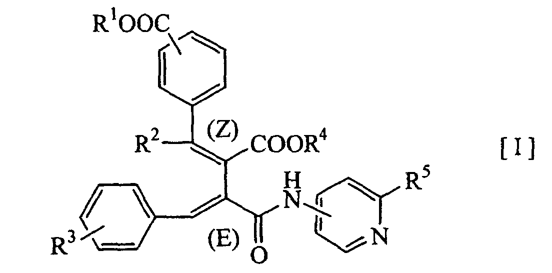 wherein R¹ is a hydrogen atom or a lower alkyl group, R² is a lower alkyl group, R³ is a lower alkoxy group, R⁴ is a hydrogen atom or a lower alkyl group and R⁵ is a lower alkyl group, or a pharmaceutically acceptable salt thereof, or a process for preparing the same.

### DETAILED DESCRIPTION OF THE PREFERRED EMBODIMENTS

Among the compounds of the present invention represented by the formula [I] above, those wherein R¹ is a hydrogen atom, R² is a methyl group, R³ is a methoxy group, R⁴ is a methyl group and R⁵ is a methyl group are preferred.

More preferred compound among the present compounds of the formula [I] is (1Z,3E)-1-methyl-1-(4-carboxyphenyl)-2-methoxycarbonyl-3-(2-methylpyridin-4-yl)aminocarbonyl-4-(3-methoxyphenyl)butadiene or a pharmaceutically acceptable salt thereof.

Furthermore, a carboxylic acid compound of the general formula [II]: 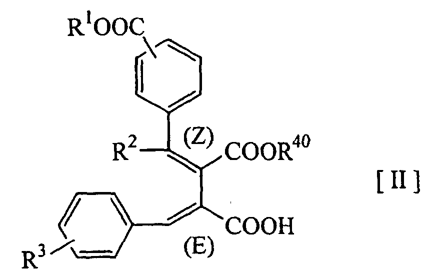 wherein R⁴⁰ is a lower alkyl group and the rest of the symbols have the same meaning as defined above, or a salt thereof, which is a synthetic intermediate of the present compound [I], is also novel.

When a compound [I] or its synthetic intermediate [II] of the present invention has an asymmetric carbon atom(s) at the substituent in groups R¹, R², R³, R⁴, R⁴⁰ and/or R⁵, it may exist in any forms of plural stereoisomers (diastereoisomers, enantiomers) owing to said asymmetric carbon atom(s), and the present invention also includes both any one of these stereoisomers or a mixture thereof.

The butadiene derivative [I] of the present invention and a pharmaceutically acceptable salt thereof having an excellent inhibitory activity on the PAI-1 production and hence are contemplated as being useful in the prophylaxis or treatment of myocardial infarction, intra-atrial thrombus in atrial fibrillation, arterial sclerosis such as atherosclerosis, angina pectoris, stroke, pulmonary infarction, deep venous thrombosis (DVT), disseminated intravascular coagulation syndrome (DIC), diabetic complications, restenosis after percutaneous transluminal coronary angioplasty (PTCA), venous embolism after high risk operations such as orthopedic surgery or abdominal surgery, acute coronary symptoms (unstable angina, non Q wave myocardial infarction), venous thrombus in cancer patients, cerebral embolism from atrial fibrillation, hepatic venous occlusion after hematopoietic stem cell transplantation, pulmonary fibrosis, peritoneal sclerosis in a patient undergoing peritoneal dialysis, septic shock, rejection after organ (kidney, etc.) transplantation, cancer metastasis, and the like. The present compound [I] and a pharmaceutically acceptable salt thereof also have antiinflammatory effects. In addition, the present compound [I] shows excellent bioavailability and stability. In addition, the present compound [I] characteristically shows less toxicity and high safety when used as a medicament. Its advantageous feature is especially dominant in regard to toxicity, showing extremely low inhibiting activity (toxicity) in the inhibitory test for liver microsome P450. It is also advantageous regarding toxic effects on chromosome.

The compound [I] of the present invention can be clinically used either in the free form or in the form of a pharmaceutically acceptable salt thereof. The pharmaceutically acceptable salt of the compound [I] includes a salt with an inorganic acid such as hydrochloride, sulfate, phosphate or hydrobromide, or a salt with an organic acid such as acetate, fumarate, oxalate, citrate, methanesulfonate, benzenesulfonate, tosylate or maleate. Besides, when the compound [I] has a substituent(s) such as carboxyl group, examples of the pharmaceutically acceptable salt include salts with a base (e.g., alkaline metal salt such as sodium salt, potassium salt or alkaline earth metal salt such as calcium salt).

The present compound [I] or a salt thereof includes either intramolecular salt or an additive thereof, and solvates or hydrates thereof.

The present compound [I] or a pharmaceutically acceptable salt thereof can be administered either orally or parenterally, and can be formulated into a conventional pharmaceutical preparation such as tablets, granules, capsules, powders, injections or inhalants.

The dose of the compound [I] of the present invention or a pharmaceutically acceptable salt thereof may vary depending on the administration rout, and the age, weight and condition of the patient. For example, the dosage as an injectable preparation can be usually in the range of about 0.01 to 10 mg/kg/day, preferably in the range of about 0.05 to 5 mg/kg/day, and the dosage for oral administration can be usually in the range of about 0.1 to 100 mg/kg/day, preferably in the range of 0.5 to 50 mg/kg/day.

### BEST MODE FOR CARRYING OUT THE INVENTION

### <Preparation of Butadiene Derivatives>

According to the present invention, the butadiene derivatives of the present invention can be prepared by the following Processes A to C, although the present invention should not be limited thereto.

### [Process A]

Among the present compound [I], a compound wherein R⁴ is a lower alkyl group, that is, a butadiene derivative of the general formula [I-a]: 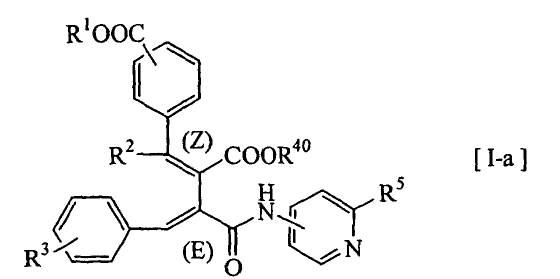 wherein R⁴⁰ is a lower alkyl group and the rest of the symbols have the same meaning as defined above can be prepared by reacting a carboxylic acid compound of the formula [II]: 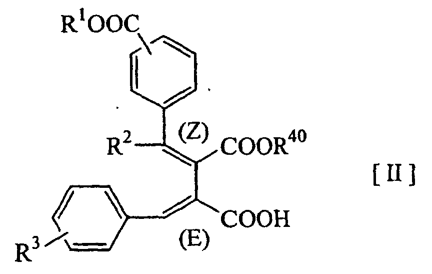 wherein each symbol has the same meaning as defined above, or a salt or a reactive derivative thereof (e.g., acid halides, mixed acid anhydrides, etc.) with an amine compound of the general formula [III]: 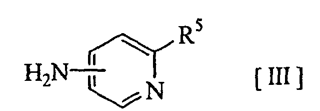 wherein each symbol has the same meaning as defined above, or a salt thereof.

The reaction between a carboxylic acid compound [II] and an amine compound [III] can be carried out in a solvent in the presence or absence of a condensing agent, a base and an activating agent. Examples of the condensing agent include, for example, dicyclohexylcarbodiimide (DCC), 1-ethyl-3-(3-dimethylaminopropyl)carbodiimide hydrochloride (WSC · HCl), diphenylphosphoryl-azide (DPPA), carbonyldiimidazole (CDI), diethylcyanophosphonate (DEPC), diisopropylcarbodiimide (DIPCI), benzotriazol-1-yloxytripyrrolidinophosphonium hexafluorophosphate (PyBOP) and the like. The activating agent includes, for example, 1-hydroxybenzotriazole (HOBt), hydroxysuccinimide (HOSu), dimethylaminopyridine (DMAP), 1-hydroxy-7-azabenzotriazole (HOAt), hydroxyphthalimide (HOPht), pentafluorophenol (Pfp-OH), and the like. The base includes, for example, triethylamine, diisopropylethylamine, 4-methylmorpholine, 1,8-diazabicyclo[5,4,0]-7-undecene (DBU), pyridine, 4-dimethylaminopyridine, and the like.

When the reaction is conducted between an acid halide as a reactive derivative of a carboxylic compound [II] and a compound [III], a carboxylic compound [II] is first converted into an acid halide using a conventional halogenating agent (e.g., thionyl chloride, oxalyl chloride, phosphoryl chloride, phosphorus trichloride, phosphorous tribromide, etc.), and the resultant acid halide is then reacted with an amine [III] in a solvent in the presence or absence of a base. The base includes, for example, triethylamine, diisopropylethylamine, 4-methylmorpholine, 1,8-diazabicyclo[5,4.0]-7-undecene (DBU), pyridine and 4-dimethylaminopyridine.

When the reaction is conducted between a mixed acid anhydride as a reactive derivative of a carboxylic compound [II] and a compound [III], a carboxylic compound [II] is first reacted with an acid halide derivative such as a chlorocarbonate ester (e.g., ethyl chlorocarbonate, isobutyl chlorocarbonate), a sulfonyl halide derivative (e.g., methanesulfonyl chloride, p-toluenesulfonyl chloride) or the like in the presence of a base (e.g., triethylamine, pyridine) to convert into a mixed acid anhydride, and the resultant mixed acid anhydride is reacted with an amine [III] in a solvent in the presence or absence of a base. The base includes, for example, triethylamine, diisopropylethylamine, 4-methylmorpholine, 1,8-diazabicyclo[5,4.0]-7-undecene (DBU), pyridine and 4-dimethylaminopyridine.

Any solvent can be used in the reactions above as far as it does not adversely affect the reaction, and examples of a solvent include dichloromethane, chloroform, N,N-dimethylformamide, N,N-dimethylacetamide, tetrahydrofuran, toluene, benzene, 1,2-dichloroethane, 1-methylpyrrolidinone, ethyl acetate, and a mixture thereof.

### [Process B]

Among the present compound [I], a compound wherein R¹ is a hydrogen atom, that is, a butadiene derivative of the general formula [I-c]: 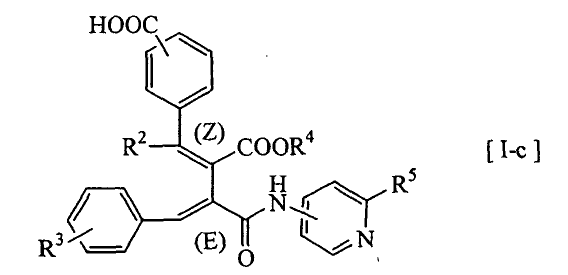 wherein each symbol has the same meaning as defined above can be prepared by treating a compound of the formula [I-b]: 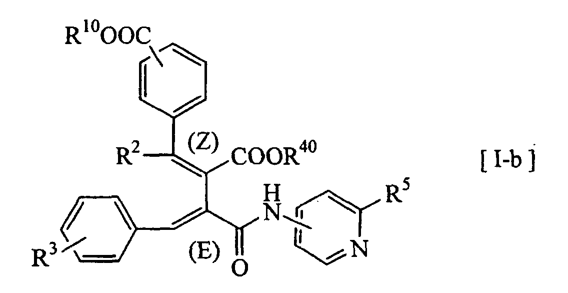 wherein R¹⁰ is a lower alkyl group and the rest of the symbols have the same meaning as defined above, or a salt thereof with an acid or a base.

This reaction can be carried out in a solvent in the presence of an acid or a base. Any solvent can be used as far as it does not adversely affect the reaction, and examples of a solvent include chloroform, dichloromethane, tetrahydrofuran, dioxane, methanol, ethanol, water, and a mixture thereof. The acid includes, for example, hydrochloric acid, sulfuric acid, trifluoroacetic acid, HCl-dioxane, HCl-methanol, p-toluenesulfonic acid, methanesulfonic acid, and the like. The base includes, for example, sodium hydroxide, potassium hydroxide, lithium hydroxide, and the like.

### [Process C]

Among the present compound [I], a compound wherein R⁴ is a hydrogen atom, that is, a butadiene derivative of the general formula [I-d]: 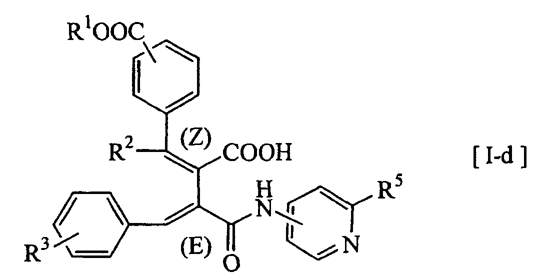 wherein each symbol has the same meaning as defined above can be prepared by treating a compound of the formula [I-b]: 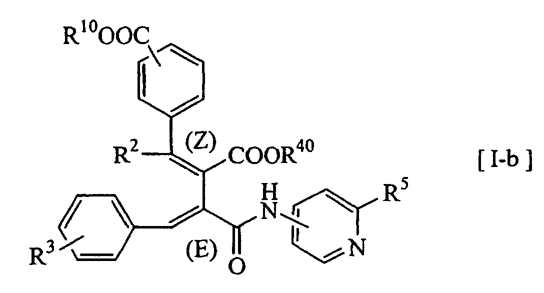 wherein each symbol has the same meaning as defined above, or a salt thereof with an acid or a base.

This reaction can be carried out in a solvent in the presence of an acid or a base. Any solvent can be used as far as it does not adversely affect the reaction, and those illustrated as usable in [Process B] above are available. Further, an acid or a base illustrated in [Process B] above are also available.

### <Manufacturing Process for Starting compound [II]>

The starting compound [II] can be prepared, for example, according to the following reaction scheme. 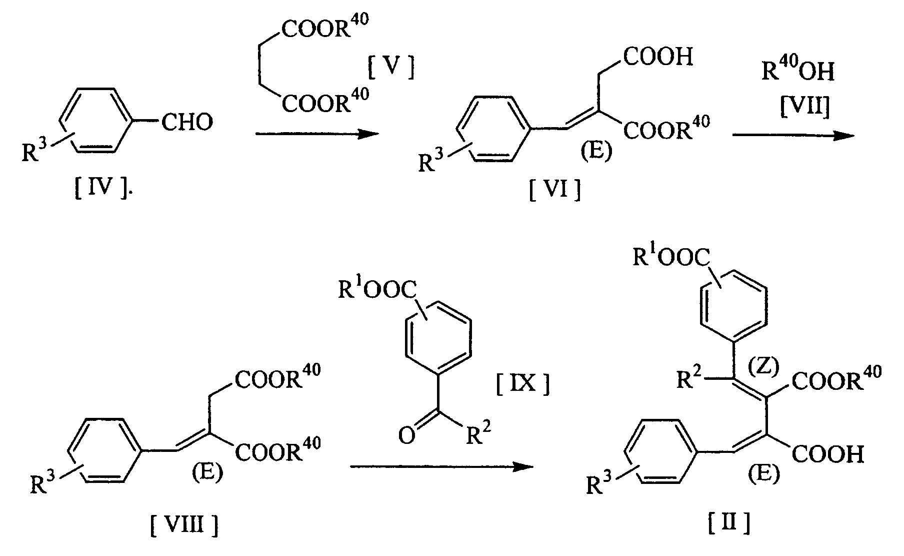 wherein each symbol has the same meaning as defined above.

The reaction wherein a compound [VI] is prepared from a compound [IV] and a compound [V] can be carried out under an ordinary condition for Stobbe Reaction, that is, in a solvent in the presence of a base. Any solvent can be used as far as it does not adversely affect the reaction, and examples of a solvent include methanol, ethanol, isopropyl alcohol, tert-butyl alcohol, benzene, toluene, xylene, tetrahydrofuran, and the like. The base includes, for example, an organic base such as potassium tert-butoxide, sodium methoxide and sodium ethoxide, or an inorganic base such as sodium hydroxide, potassium hydroxide, and the like.

The reaction wherein a compound [VIII] is prepared from a compound [VI] and a compound [VII] can be carried out under conventional conditions for esterification reaction. For example, it can be carried out by a method wherein a compound [VI] is reacted with a lower alcohol [VII] in the presence of an acid catalyst (for example, conc. sulfuric acid, conc. hydrochloric acid, polyphosphoric acid, etc.), or a method wherein a compound [VI] is converted into an acid halide by halogenating the carboxylic acid with a halogenating agent (e.g., thionyl chloride, oxalyl chloride), and then reacted with a lower alcohol [VII] in the presence of a base (for example, triethylamine, pyridine, etc.).

The reaction wherein a compound [II] is prepared from a compound [VIII] and a compound [IX] can be carried out under an ordinary condition for Stobbe Reaction. As to the solvent and the base, those illustrated above as usable in regard to the production of a compound [VI] from a compounds [IV] and [V] are available.

### <Manufacturing Process for Starting compound [III]>

The starting compound [III] can be prepared, for example, according to the following reaction scheme. 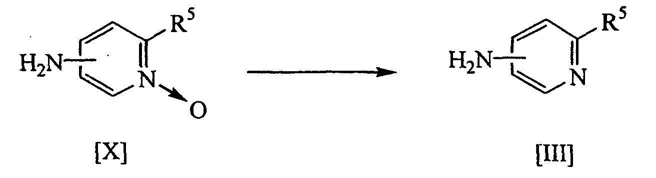 wherein each symbol has the same meaning as defined above.

The reaction wherein a compound [III] is prepared from a compound [X] can be carried out in a solvent in the presence of a reducing agent. Any solvent can be used as far as it does not adversely affect the reaction, and examples of a solvent include acetic acid, methanol, ethanol, dioxane, tetrahydrofuran, and the like. The reducing agent includes, for example, palladium-carbon/hydrogen, palladium-carbon/formic acid, Raney nickel/hydrogen, lithium aluminum hydride and sodium borohydride. The present reaction can be conducted at normal pressures or under pressure.

In the above-mentioned processes for preparing respective objective compounds, not only the intermediates illustrated in the reaction schemes above but also salts or reactive derivatives thereof, which do not adversely affect the reaction, are available,

Furthermore, in the preparation of a compound of the present invention and starting compounds thereof, when the respective starting or intermediate compounds have a functional group(s), they can be used either in the form as illustrated above or in the protected from. In the latter case, an appropriate protecting group can be introduced and removed when it becomes unnecessary by a conventional method of synthetic chemistry.

When used herein in regards to the compounds of the present invention and the starting compounds, the term "alkyl group" means a straight- or branched-chain alkyl group having 1 to 16 carbon atoms, preferably 1 to 8 carbon atoms. The term "lower alkyl group" or "lower alkoxy group" means a straight- or branched-chain alkyl or alkoxy group having 1 to 6 carbon atoms, preferably 1 to 4 carbon atoms. The term "halogen atom" means fluorine, chlorine, bromine or iodine atom.

The following Examples are provided to further illustrate the present invention and should not be construed as limiting the scope of the present invention.

### Example 1

(1) 4-Acetylbenzoic acid (100 g) is suspended into 500 ml of dichloromethane. To the suspension are added 64 ml of oxalyl chloride and 4.7 ml of N,N-dimethylformamide at room temperature, and the mixture is stirred for 2 hours at room temperature. The reaction solution is concentrated under reduced pressure and the residue is dissolved by adding 250 ml of chloroform and 175 ml of tert-butyl alcohol. To the solution is then added dropwise 156 ml of pyridine under ice-cooling (12°C). The reaction mixture is stirred for 30 minutes at room temperature and concentrated under reduced pressure. The residue is dissolved in toluene and the solution is washed with 2M hydrochloric acid, saturated aqueous sodium hydrogen carbonate solution and brine, successively. The organic layer is dried over sodium sulfate and concentrated under reduced pressure. Hexane is added to the residue and the mixture is stirred under ice-cooling. The precipitated crystals are collected by filtration, washed with cold hexane and dried in vacuo to give tert-butyl 4-acetylbenzoate (yield 96.82 g, 69 %).
   M.p. 59-60 °C
(2) To a solution of dimethyl succinate (773 g) in 600 ml of methanol is added sodium methoxide/methanol solution (28 %, 850 ml), and the mixture is heated to reflux for 30 minutes. The reaction solution is cooled to 65 °C and a solution of 600 mg of m-anisaldehyde in methanol (300 ml) is added dropwise over 30 minutes. The reaction solution is heated to reflux for 3 hours, and concentrated under reduced pressure. The residue is dissolved in 2 L of water and washed with chloroform (1.5 L x 2). To the aqueous layer is added dropwise 441 ml of conc. hydrochloric acid with stirring under ice-cooling and the mixture is extracted with 4 L of ethyl acetate, washed with 3 L of brine and dried over sodium sulfate. The organic layer is concentrated under reduced pressure to give the residue as a crude carboxylic acid compound (1075 g). The residue is dissolved in 2.4 L of methanol, combined with 24.5 ml of conc. sulfuric acid, heated under refluxing for 3 hours and concentrated under reduced pressure. To the residue is added 4 L of ethyl acetate, followed by washing with water (3 L), aqueous saturated sodium hydrogen carbonate solution (3L x 3) and brine (3 L), successively. The organic layer is dried over magnesium sulfate and concentrated under reduced pressure. The residue is distilled in vacuo (Bp. 145-150 °C / 1 mmHg) to give dimethyl (E)-2-(3-methoxybenzylidene)-succinate (yield 747 g, 64 %) as pale yellow oil.
(3) 2-Methyl-4-nitropyridine-N-oxide (30 g) is dissolved in 330 ml of acetic acid. The solution is treated under catalytic hydrogenation conditions under pressure with Parr in the presence of 20 g of 10 % Pd/C (wet), and, 24-hour later, bubbled with nitrogen for 30 minutes followed by removal of Pd/C by filtration. The filtrate is concentrated to remove acetic acid and the residue is combined with aqueous saturated potassium hydrogen carbonate solution to make the mixture basic. After addition of tetrahydrofuran, the mixture is vigorously stirred and insoluble substances are removed by filtration. The organic layer is separated and the aqueous layer is extracted with tetrahydrofuran (x2). The combined organic layer is then concentrated in vacuo. To the residue is added toluene, and the mixture is subjected to azeotropic evaporation to remove water to obtain precipitated solids. The solids are dissolved in dichloromethane, and the solution is dried over magnesium sulfate and concentrated under reduced pressure. The precipitated crystals are triturated with diisopropyl ether and collected by filtration. The resultant crystals are air dried at 50 °C overnight to give 4-amino-2-methylpyridine (yield 18.7 g, 88 %).
   Mp. 86-88 °C
(4) The compound (10.0 g) obtained in (2) above and the compound (8.33 g) obtained in (1) above are dissolved in 55 ml of tert-butyl alcohol. To the solution, a solution of 4.67 g of potassium tert-butoxide in tert-butyl alcohol (40 ml) is added dropwise (under nitrogen atmosphere, ice-cooling, internal temperature of 21-23°C), which is followed by stirring for 2 hours at 20 °C. To the reaction solution is added 0.87 ml of acetic acid, and the mixture is concentrated under reduced pressure. The residue is mixed with toluene, subjected to azeotropic evaporation, and the residue is re-dissolved in 100 ml of toluene. To the solution is added 1.69 g of potassium tert-butoxide under ice-cooling, and stirred for 1 hour at 20 °C. After addition of 2.8 ml of acetic acid, the organic layer is washed with brine, 1 M hydrochloric acid, water and brine, successively, dried over magnesium sulfate and concentrated under reduced pressure. The residue is dissolved in 200 ml of diisopropyl ether. To the solution is added dropwise 5.1 g of 28 % sodium methoxide solution in methanol under ice-cooling, and stirred at room temperature overnight. The precipitated crystals are collected by filtration, washed with diisopropyl ether and air-dried (50 °C) to give sodium (1Z, 3E)-1-methyl-1-(4-t-butoxycarbonylphenyl)-2-methoxy-carbonyl-4-(3-methoxyphenyl)butadiene-3-carboxylate (yield 6.58 g, 37 %).
   MP. > 196 °C (decomp.)
(5) The compound (152 g) obtained in (4) above is suspended in chloroform. To the suspension are added 37.8 ml of oxalyl chloride and 2.51 ml of 4-dimethylformamide, and the mixture is stirred for 1 hour at 50 °C. The reaction solution is concentrated under reduced pressure, and the residue, after addition of chloroform, is concentrated again under reduced pressure. The residue is combined with 1.5 L of tetrahydrofuran, and a solution of 41.7 g of the compound obtained in (3) above in 200 ml of tetrahydrofuran is added dropwise with stirring under ice-cooling. After addition of 3.96 g of 4-dimethylaminopyridine at the same temperature, 67.2 ml of triethylamine is added dropwise (internal temperature 7-10 °C). The reaction solution is stirred for 40 minutes at room temperature and concentrated under reduced pressure. The residue is dissolved in ethyl acetate, and the solution is washed with brine (x3), dried over magnesium sulfate, and concentrated under reduced pressure to give (1Z, 3E)-1-methyl-1-(4-t-butoxycarbonyl-phenyl)-2-methoxycarbonyl-3-(2-methylpyridin-4-yl)aminocarbonyl-4-(3-methoxyphenyl)butadiene as a crude product.
(6) To the crude product obtained in (5) above are added 300 ml of chloroform and 1 L of 1.4 M hydrogen chloride solution in dioxane and the mixture is stirred at room temperature overnight. The precipitated crystals are collected by filtration, washed with tetrahydrofuran, and the crude crystals are purified by silica gel column chromatography (eluent; chloroform:methanol = 20:1), and recrystallized from methanol to give (1Z,3E)-1-methyl-1-(4-carboxyphenyl)-2-methoxycarbonyl-3-(2-methylpyridin-4-yl)aminocarbonyl-4-(3-methoxyphenyl)butadiene hydrochloride (yield 86.4 g, 51 %).
   Mp. 232 °C (decomp.)

## Claims

1. A butadiene derivative of the general formula [I]: 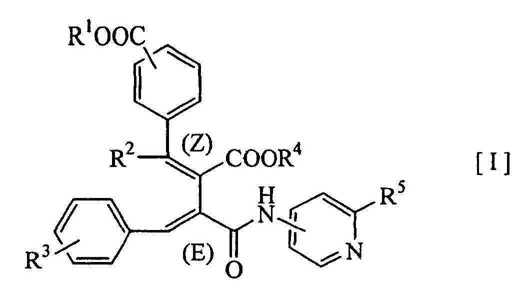 wherein R¹ is a hydrogen atom or a lower alkyl group, R² is a lower alkyl group, R³ is a lower alkoxy group, R⁴ is a hydrogen atom or a lower alkyl group, R⁵ is a lower alkyl group, or a pharmaceutically acceptable salt thereof.

2. The compound according to claim 1 wherein R¹ is a hydrogen atom, R² is a methyl group, R³ is a methoxy group, R⁴ is a methyl group and R⁵ is a methyl group.

3. (1Z,3E)-1-Methyl-1-(4-carboxyphenyl)-2-methoxycarbonyl-3-(2-methylpyridin-4-yl)aminocarbonyl-4-(3-methoxyphenyl)butadiene or a pharmaceutically acceptable salt thereof.

4. A process for preparing a butadiene derivative of the general formula [I-a]: 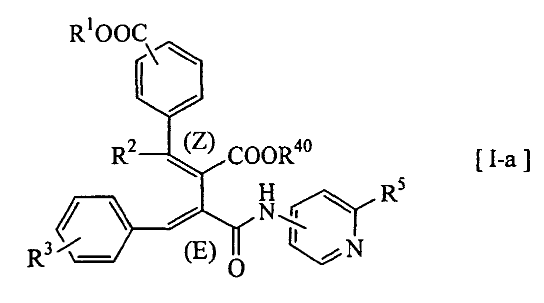 wherein R¹ is a hydrogen atom or a lower alkyl group, R² is a lower alkyl group, R³ is a lower alkoxy group, R⁵ is a lower alkyl group and R⁴⁰ is a lower alkyl group, or a pharmaceutically acceptable salt thereof, which comprises reacting a carboxylic acid compound of the formula [II]: 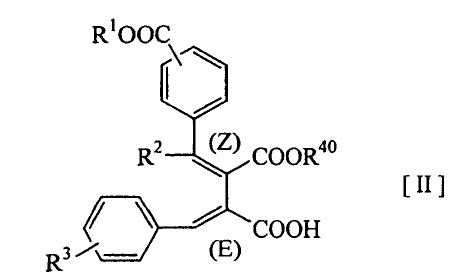 wherein each symbol has the same meaning as defined above, or a salt or a reactive derivative thereof with an amine compound of the general formula [III]: 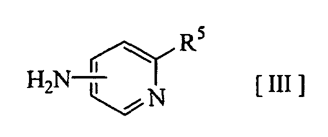 wherein R⁵ has the same meaning as defined above, or a salt thereof, and if necessary, followed by converting the product into a pharmaceutically acceptable salt thereof.

5. A process for preparing a butadiene derivative of the general formula [I-c]: 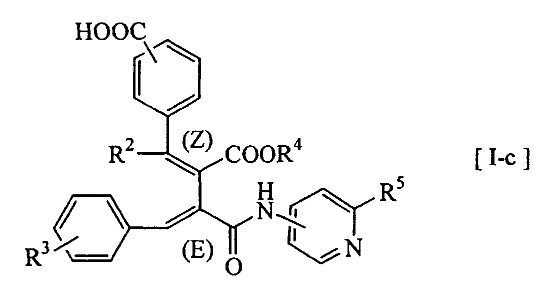 wherein R² is a lower alkyl group, R³ is a lower alkoxy group, R⁴ is a hydrogen atom or a lower alkyl group and R⁵ is a lower alkyl group, or a pharmaceutically acceptable salt thereof, which comprises treating a compound of the general formula [I-b]: 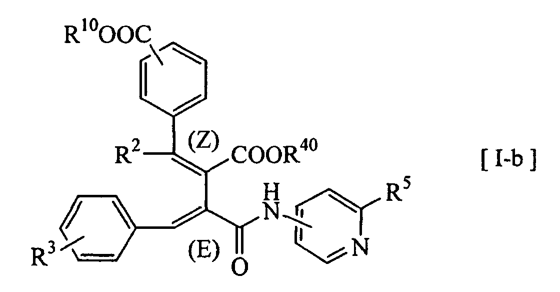 wherein R¹⁰ is a lower alkyl group, R⁴⁰ is a lower alkyl group and the rest of the symbols have the same meaning as defined above, or a salt thereof with an acid or a base, and if necessary, followed by converting the product into a pharmaceutically acceptable salt thereof.

6. A carboxylic acid compound of the general formula [II]: 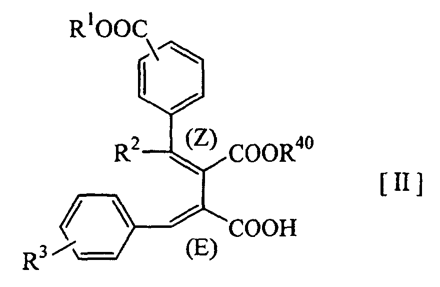 wherein R¹ is a hydrogen atom or a lower alkyl group, R² is a lower alkyl group, R³ is a lower alkoxy group and R⁴⁰ is a lower alkyl group, or a salt thereof.

7. A pharmaceutical composition, which comprises as an active ingredient a compound according to any one of claims 1 to 3 or a pharmaceutically acceptable salt thereof.

8. A method for the treatment of venous embolism after high risk operations such as orthopedic surgery or abdominal surgery, which comprises administering an effective amount of a compound according to any one of claims 1 to 3 or a pharmaceutically acceptable salt thereof to a patient in need thereof

9. A method for the treatment of cerebral embolism from atrial fibrillation, which comprises administering an effective amount of a compound according to any one of claims 1 to 3 or a pharmaceutically acceptable salt thereof to a patient in need thereof.

10. A method for the treatment of acute coronary symptoms, which comprises administering an effective amount of a compound according to any one of claims 1 to 3 or a pharmaceutically acceptable salt thereof to a patient in need thereof.

11. A method for the treatment of restenosis after percutaneous transluminal coronary angioplasty (PTCA), which comprises administering an effective amount of a compound according to any one of claims 1 to 3 or a pharmaceutically acceptable salt thereof to a patient in need thereof.

12. Use of a compound according to any one of claims 1 to 3 or a pharmaceutically acceptable salt thereof for treating a patient of venous embolism after high risk operations such as orthopedic surgery or abdominal surgery.

13. Use of a compound according to any one of claims 1 to 3 or a pharmaceutically acceptable salt thereof for treating a patient of cerebral embolism from atrial fibrillation.

14. Use of a compound according to any one of claims 1 to 3 or a pharmaceutically acceptable salt thereof for treating a patient of acute coronary symptoms.

15. Use of a compound according to any one of claims 1 to 3 or a pharmaceutically acceptable salt thereof for treating a patient of restenosis after percutaneous transluminal coronary angioplasty (PTCA).
